# EUROPEAN PATENT APPLICATION

(11) **EP 0 962 207 A2**
(43) Date of publication of application: **08.12.1999**
(21) Application number: 99304284.5
(22) Date of filing: 01.06.1999
(51) Int. Cl.: A61F 13/15

(54) **Disposable diaper**

(30) Priority: 30.05.1998 JP 18804998
(71) Applicant: UNI-CHARM CORPORATION, Kawanoe-shi Ehime-ken (JP)
(72) Inventor: Suekane, Makoto, Research & Development Division, Mitoyo-gun, Kagawa-ken 769-1602 (JP)
(74) Representative: Kensett, John Hinton

(57) **Abstract**

A disposable diaper having a cover sheet 200 lying above a topsheet 2 thereof, the cover sheet being formed in a transversely middle zone thereof with an opening 14, the opening 14 being surrounded by a first elastic member 31 and a second elastic member 32, the first and second elastic members 31, 32 intersecting each other in proximity of the opening 14 and further extending respective halves of legsurrounding side edges and the other halves of the legsurrounding side edges.

## Description

The present invention relates to a disposable diaper for absorption and containment of body wastes.

It is well known to provide a cover sheet above a liquid-pervious topsheet of a disposable diaper and to form the cover sheet with an opening lying above a crotch region of the diaper wherein a periphery of the opening is at least partially provided with elastic members.

For example, Japanese Patent Application Disclosure Gazette (Kokai) No. Sho61-41304 discloses a disposable diaper having a cover sheet which is formed with a longitudinally larger opening provided along its transversely opposite side edges with stretchable/contractable elastic members extending rectilinearly.

Japanese Patent Application Disclosure Gazette (Kokai) No. Hei5-293138 discloses a disposable diaper having a cover sheet formed with an elastic opening, in which the opening of the cover sheet is provided along its entire periphery with elastic members.

Supposed that the diaper is put on a wearer's body with such opening surrounding the wearer's anus and urinary organs within its coverage area so as to receive body wastes in a space defined between the cover sheet and the topsheet, leakage of body waste will be avoided and the wearer's skin will be protected from being soiled with body wastes. Additionally, if the opening expected to function in this manner has its periphery adapted to be elastically stretchable/contractable, the periphery will be easily fitted to the wearer's skin and ensure body wastes to be trapped in the foresaid space. However, it is generally not easy to provide the opening along its entire periphery using mechanical means.

In the case of the disposable diaper described in said Japanese Patent Application Disclosure Gazette (Kokai) No. Sho61-41304, only a part of the opening periphery can be deformed and placed in close contact with the wearer's skin. The remaining part of the opening periphery provided with no elastic member can not be brought in close contact with the wearer's skin and may cause a leakage of body wastes. It is impossible for the diaper to meet a need for providing the entire periphery of the opening with the elastic members.

In the case of the diaper obtained according to the method described in the Japanese Patent Application Disclosure Gazette (Kokai) No. Hei5-293138, the opening of the cover sheet is certainly provided along its entire periphery with the elastic members. However, the cover sheet comprises a pair of sheets overlapped and bonded to each other along a center line dividing a width of the diaper. With a consequence, the cover sheet to be directly placed against the wearer's skin includes a sheet-to-sheet bond line extending longitudinally of the diaper and therefore the sheet-to-sheet bond line may create a feeling of discomfort against the wearer.

In view of the problem as has been described above, it is an object of the invention to provide a disposable diaper including a cover sheet formed with an opening adapted to be elastically stretchable/contractable along its entire periphery without creating a feeling of discomfort against the wearer.

According to the invention, there is a disposable diaper comprising a basic diaper structure comprising a liquid-pervious topsheet, a liquid-impervious backsheet and a liquid-absorbent core disposed therebetween, the basic diaper structure being defined by longitudinally front and rear ends both extending circumferentially of a wearer's torso and a pair of side edges extending in parallel to each other and orthogonally to the front and rear ends, the basic diaper structure having, between the front and rear ends, a front waist region, a rear waist region and a crotch region extending therebetween, transversely opposite side edges of the crotch region being inwardly curved, and a cover sheet combined with the basic diaper structure and extending above the basic diaper structure, the cover sheet having an opening extending across above a transversely middle zone of the crotch region in the basic diaper structure into zones above the front and rear waist regions, respectively, and being provided along a periphery of the opening with stretchable/contractable elastic members so that the cover sheet is spaced from the topsheet at least along the periphery of the opening and bonded to an inner surface of the basic diaper structure along an outer periphery thereof.

In such disposable diaper, the present invention is characterized in that the elastic members comprise a first elastic member extending along a half of the periphery of the opening curved toward the front waist region and a second elastic member extending along another half of the periphery of the opening curved toward the rear waist region, the first and second elastic members intersect each other in proximity of the periphery of the opening, the first elastic member further extending along halves of side edges of the crotch region and the second elastic member further extending along the other halves of the side edges of the crotch region.
Fig. 1 is a plan view of a disposable diaper according to the present invention;
Fig. 2 is a sectional view taken along a line II-II in Fig. 1;
Fig. 3 is a sectional view taken along a line III-III in Fig. 1;
Fig. 4 is a view similar to Fig. 3 showing a basic diaper structure as being curved;
Fig. 5 is a view similar to Fig. 3 showing an alternative embodiment of the present invention; and
Fig. 6 is a schematic diagram illustrating the steps of making a cover sheet.

Details of a disposable diaper according to the present invention will be more fully understood from the description given hereunder with reference to the accompanying drawings.

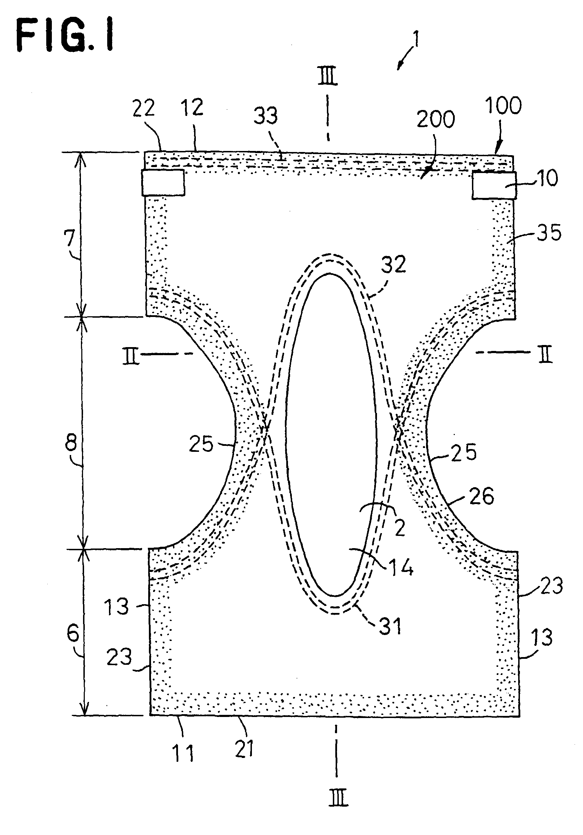

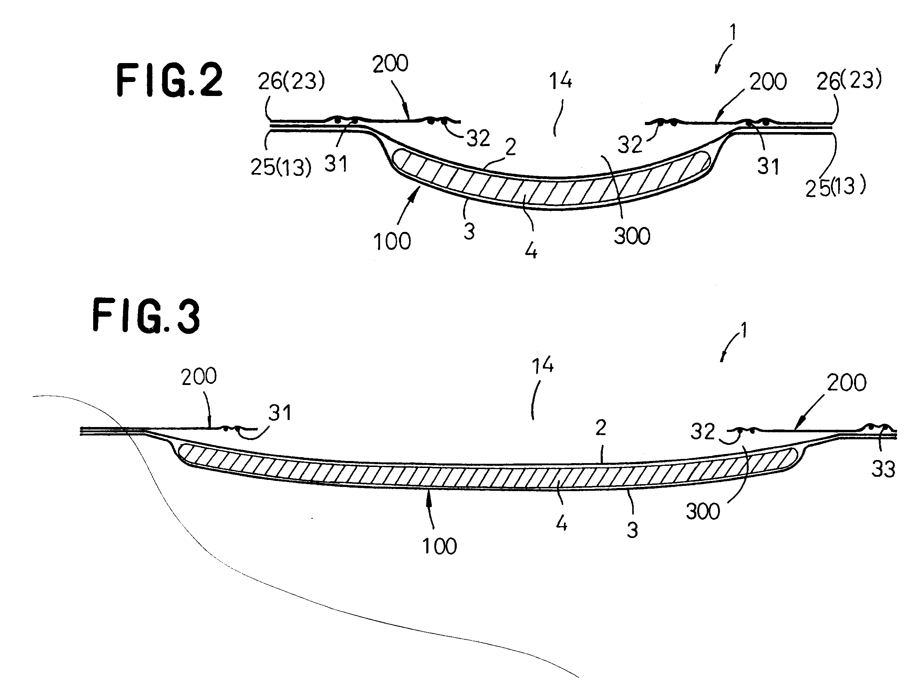

Fig. 1 is a plan view of a disposable diaper 1, and Figs. 2 and 3 are sectional views taken along lines II-II and III-III in Fig. 1, respectively.

The diaper 1 comprises a basic diaper structure 100 and a cover sheet 200 attached to an upper surface of the basic diaper structure 100. The basic diaper structure 100 is generally hourglass-shaped and comprises a liquid-pervious topsheet 2, a liquid-impervious backsheet 3 and a liquid-absorbent core 4 disposed between these two sheets 2, 3. Respective portions of the topsheet 2 and the backsheet 3 extending outward beyond a peripheral edge of the core 4 are placed upon and bonded to each other. The diaper 1 has front and rear ends 11, 12 extending transversely of the diaper 1 and a pair of side edges 13, 13 extending longitudinally of the diaper 1, i.e., orthogonally to the front and rear ends 11, 12. Configurationally, the diaper 1 is longitudinally composed of a front waist region 6, a rear waist region 7 and a crotch region 8 extending between these two waist regions 6, 7. In the crotch region 8, the side edges 13, 13 are curved inward to define circular-arc-shaped side edges 25 adapted to surround a wearer's legs.

The cover sheet 200 is preferably formed of a liquid-resistant material and contoured substantially the same as the basic diaper structure 100 and has front and rear ends 21, 22 and a pair of side edges 23, 23. In the crotch region 8 of the basic diaper structure 100, the side edges 23, 23 are curved inward to define generally circular-arc-shaped side edges 26, 26 adapted to surround a wearer's legs. The cover sheet 200 is formed in a transversely middle zone with an opening 14 extending across the crotch region 8 of the basic diaper structure 100 into the front and rear waist regions 6, 7 so that the topsheet 2 of the basic diaper structure 100 may be partially seen through the opening 14 (See Fig. 1). A first elastic member 31, a second elastic member 32 and a third elastic member 33 are secured in a stretched state to an inner surface of the cover sheet 200 facing the topsheet 2. The first elastic member 31 extends along approximately a half of the periphery of the opening 14 curved toward the front waist region 6 and further extends along the respective halves of the leg surrounding side edges 26 which extend in the crotch region 8 a little into the rear waist region 7. Similarly, the second elastic member 32 extends along approximately the other half of the periphery of the opening 14 curved toward the rear waist region 7 and further extends along the respective other halves of the leg surrounding side edges 26 which extend in the crotch region 8 a little into the front waist region 6. The first and second elastic members 31, 32 intersect each other in proximity of the opening 14 so that the cover sheet 200 may be elastically stretchable not only along a full periphery of the opening 14 but also along respective full lengths of the leg surrounding side edges 26 and portions of the elastic members 31, 32 respectively positioned along the peripheries of the opening 14 and the leg surrounding side edges 26 function cooperatively with each other. The third elastic member 33 extends in parallel to the rear end 22 so that the cover sheet 200 may be elastically stretchable around a wearer's waist.

The cover sheet 200 is bonded to an inner surface of the basic diaper structure 100 (to the topsheet 2 if the inner surface of the basic diaper structure 100 is defined by the topsheet 2 as in the illustrated embodiment) at least along the front and rear ends 11, 12 and the side edges 13, 13 of the basic diaper structure 100. As will be obvious, the first and second elastic members 31, 32 previously secured to the cover sheet 200 must be maintained in their stretched states when the cover sheet 200 is bonded to the topsheet 2. In this manner, leakage of body wastes is reliably avoided. Referring to Fig. 1, an area over which the cover sheet 200 is bonded to the topsheet 2 is indicated by a plurality of dots 35. As will be apparent from Fig. 1, regions in proximity of the first and second elastic members 31, 32 extending along the leg surrounding side edges 26 must be included in the bonding area when the leg surrounding side edges 26 of the cover sheet 200 are bonded to the basic diaper structure 100. On the other hand, a peripheral region of the opening 14 having the first and second elastic members 31, 32 bonded thereto should be excluded from the foresaid area over which the cover sheet 200 is bonded to the basic diaper structure 100. Bonding the cover sheet 200 to the basic diaper structure 100 in this manner enables the diaper 1 to have an elastic stretchability fully along the periphery of the opening 14, fully along the leg surrounding side edges 26, 26 and in proximity of the end of the rear waist region 7.

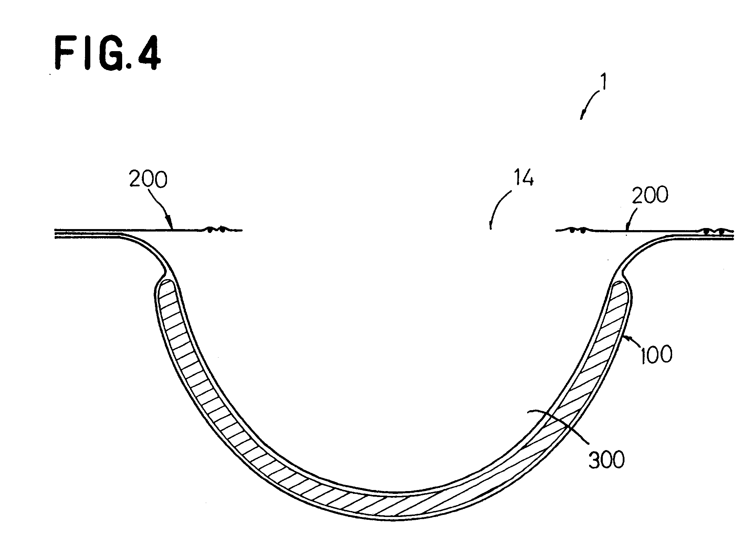

Fig. 4 is a view similar to Fig. 3 showing the diaper 1 with its basic diaper structure 100 being curved downward when the diaper 1 is put on a wearer's body. Now it is supposed that the diaper 1 is put on a wearer's body so that the diaper wearer's anus and urinary organs lie within a coverage area of the opening 14. The basic diaper structure 100 is curved with the cover sheet 200 lying inside as shown and, in the peripheral region of the opening 14, the cover sheet 200 is lifted off from the basic diaper structure 100 as the first and second elastic members 31, 32 contract. Thus, the peripheral region comes in close contact with the wearer's skin and thereby a space 300 is formed between the cover sheet 200 and the basic diaper structure 100. In this state, the diaper 1 can reliably receive and hold body wastes within the coverage area of the opening 14.

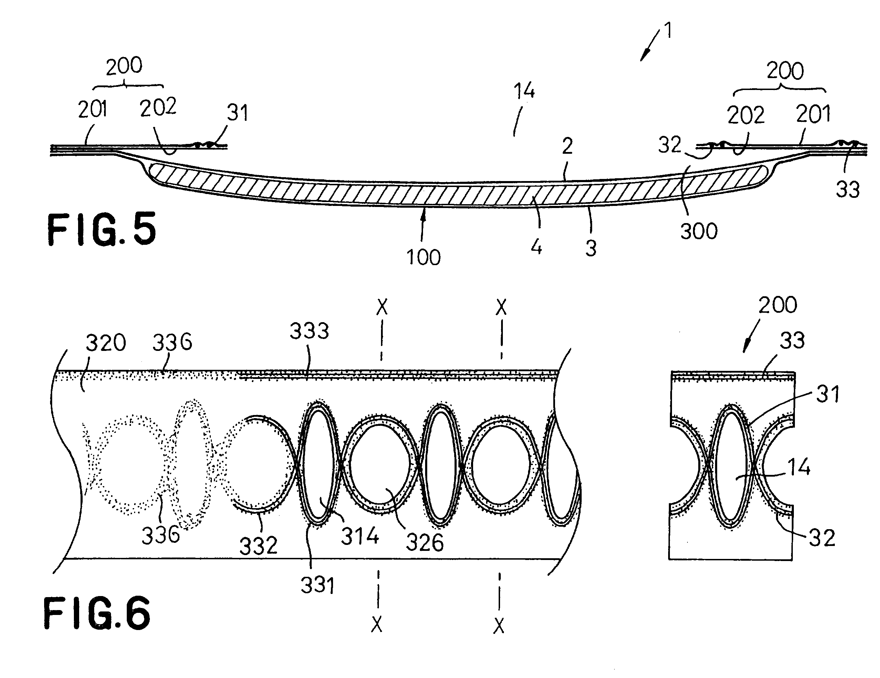

Fig. 5 is a view similar to Fig. 3, showing an alternative embodiment of the present invention. With the diaper 1 according to this embodiment, the cover sheet 200 comprises an outer layer sheet 201 and an inner layer sheet 202 bonded to each other. The first, second and third elastic members 31, 32, 33 are disposed between the outer and inner layer sheets 201, 202 and secured in a stretched state to an inner surface of at least one of these two layer sheets 201, 202. The cover sheet 200 of such an arrangement may use a nonwoven fabric as the outer layer sheet 201 and a nonwoven fabric or a plastic film as the inner layer sheet 202 to obtain the cover sheet 200 which offers a soft touch and high liquid-impermeability.

Fig. 6 is a diagram schematically illustrating a method for continuously making the cover sheet used in the diaper 1 of Fig. 1. As illustrated, a web 320 for forming the cover sheet 200 is continuously fed from left to right as viewed in this diagram. The web 320 fed in this manner is then applied along predetermined courses on its one surface with adhesive 336. Onto this surface, elastic members 331, 332, 333 for forming the elastic members 31, 32, 33, respectively are continuously fed in a stretched state. Traverse means of well known art (not shown) are used to swing the elastic members 331, 332 so that these elastic members 331, 332 describe on the web 320 two sine curve-like lines having phases shifted from each other. The elastic member 333 is rectilinearly fed. The elastic members 331 - 333 are secured to the web by means of the adhesive 336 already applied on the web 320. Thereafter the web 320 is formed with openings 314 for forming the openings 14 and openings 326 for forming the generally circular-arc-shaped leg surrounding side edges 26, both intermittently in a machine direction for manufacturing the diaper, as will be apparent from Fig. 6. Then, the web 320 is transversely cut along lines X-X each dividing the opening 326 in two and thereby the cover sheets 200 of the individual diapers 1 are obtained. Each of the cover sheets 200 thus obtained includes the first and second elastic members 31, 32 extending along the entire periphery of the opening 14, but no sheet-to-sheet bond line which might create a feeling of discomfort against a wearer as the conventional cover sheet has been the case. The web 320 may be cut also after the web 320 has been bonded to the other web for forming the basic diaper structure 100.

For exploitation of the present invention, means to bond the respective members to each other is not limited to use of adhesive agent such as hot melt adhesive and, for the heat-sealable members, the known heat-sealing technique may be also adopted.

The cover sheet for the disposable diaper provided by the present invention is advantageous particularly in that its opening has an elasticity along the entire periphery thereof. Accordingly, such feature enables the cover sheet to be maintained in close contact along the periphery of its opening with the wearer's skin and thereby ensures body wastes to be received in the opening. In addition, this novel cover sheet is free from a sheet-to-sheet bond line and offers a good feeling to wear the diaper.

## Claims

1. A disposable diaper comprising a basic diaper structure comprising a liquid-pervious topsheet, a liquid-impervious backsheet and a liquid-absorbent core disposed therebetween, said basic diaper structure being defined by longitudinally front and rear ends both extending circumferentially of a wearer's torso and a pair of side edges extending in parallel to each other and orthogonally to said front and rear ends, said basic diaper structure having, between said front and rear ends, a front waist region, a rear waist region and a crotch region extending therebetween, transversely opposite side edges of said crotch region being inwardly curved, and a cover sheet combined with said basic diaper structure and extending above said basic diaper structure, said cover sheet having an opening extending across above a transversely middle zone of the crotch region in said basic diaper structure into zones above said front and rear waist regions, respectively, and being provided along a periphery of said opening with stretchable/contractable elastic members so that said cover sheet is spaced from said topsheet at least along the periphery of said opening and bonded to an inner surface of said basic diaper structure along an outer periphery thereof, wherein:
said elastic members comprise a first elastic member extending along a half of the periphery of said opening curved toward said front waist region and a second elastic member extending along another half of the periphery of said opening curved toward said rear waist region, said first and second elastic members intersect each other in proximity of the periphery of said opening, said first elastic member further extending along halves of side edges of said crotch region and said second elastic member further extending along the other halves of the side edges of said crotch.

2. A disposable diaper according to Claim 1, wherein said cover sheet comprises an outer layer sheet and an inner layer sheet bonded thereto.

3. A disposable diaper according to Claim 1, wherein said cover sheet is formed of a nonwoven fabric.

4. A disposable diaper according to Claim 1, wherein said outer layer sheet is formed of a nonwoven fabric and said inner layer sheet is formed of a plastic film.

5. A disposable diaper according to Claim 1, wherein said cover sheet is of liquid-resistance.

6. A disposable diaper according to Claim 1, wherein portions of said first and second elastic members respectively positioned along the peripheries of said opening and said leg surrounding side edges function cooperatively with each other.
